# EUROPEAN PATENT APPLICATION

(11) **EP 4 273 204 A1**
(43) Date of publication of application: **08.11.2023**
(21) Application number: 22020206.3
(22) Date of filing: 03.05.2022
(51) Int. Cl.: C09J 183/04, C09J 183/05, C09J 183/07, A61L 24/04

(54) **MEDICAL SILICONE PRESSURE-SENSITIVE ADHESIVE COMPOSITION**

(71) Applicant: ELKEM SILICONES France SAS, 69003 Lyon (FR)
(72) Inventor: DELOCHE, Morgane, 69560 SAINT-ROMAIN-EN-GAL (FR); MOINE, Caroline, 42290 SORBIERS (FR)
(74) Representative: Menville, Laure

(57) **Abstract**

The present invention relates to a medical silicone pressure-sensitive adhesive composition comprising at least one organopolysiloxane A comprising, per molecule, at least two C₂ to C₆ alkenyl radicals each bonded to a silicon atom, at least one organopolysiloxane resin B comprising OH group(s) bonded to silicone atoms, at least one organopolysiloxane extender CE having exactly two terminal-hydrogen atom bonded to silicone atoms, optionally at least one organopolysiloxane crosslinker XL having at least three hydrogen atoms bonded to silicone atoms, at least one hydrosilylation catalyst D, at least one solvent E, and optionally at least one hydrosilylation inhibitor F, wherein the organopolysiloxanes A, CE and XL are selected such that the molar ratio RHAlk is comprised from 2 to 5 and the ratio nHXL/nHCE is below 0.10.

## Description

### Technical field

The field of the present invention is that of a medical pressure-sensitive adhesive (PSA). Specifically, the present invention relates to a medical silicone pressure-sensitive adhesive composition, to a method for coating a substrate by using the medical silicone pressure-sensitive adhesive composition, and also to a skin-adhesive article obtainable according to the method.

### Background art

PSA is an abbreviation for the term "a pressure-sensitive adhesive", which is well known in the art and widely used in various applications, especially in medical applications. Now in the medical market, most of skin touch products such as tape, patch and bandage are prepared by acrylic-based PSA. However, acrylic-based PSA has some disadvantages such as weak sweat (water) resistance, weak biocompatibility, and high sensitization rate for human skin, especially for infants and children.

A silicone-based PSA is also widely used in medical applications for use against the skin or in contact with the skin. A silicone-based PSA is capable of adhering to a surface simply by contact or under the effect of a light pressure. It has significant advantages over acrylic-based PSA. A silicone-based PSA exhibits advantageous properties for medical applications due to its air permeability, water resistance, low irritation and biocompatibility. For example, a silicone-based PSA is suited to the enhanced requirements of novel medical applications due to its biocompatibility and its permeability, making possible the diffusion of oxygen, carbon dioxide and water vapor, which renders it perfectly suited to medical applications in which enhanced aeration is necessary.

WO2017158249A1 and WO 2017/051083 discloses skin-adhesive silicone gels. The silicone gel, due to its intrinsic natures, lacks adhesion to moist skin and thus is beneficial for wound care applications because it can avoid secondary damage to the wound during use of a dressing. However, for medical materials attached on the skin non-traumatically, a moist environment or sweating often causes the gel to lose its tack and then peels off.

WO2020099999A1 describes a medical silicone pressure-sensitive adhesive. The silicone composition is cured by poly condensation reaction: the condensation product is formed by the reaction between the polyorganosiloxane containing terminal hydroxyl groups and the silicate resin, and then a non-reactive polyorganosiloxane is added. In the subsequent tape production process, an electron beam is used to promote the crosslinking of the non-reactive polyorganosiloxane, thereby forming a layer of pressure-sensitive adhesive on the substrate. The production process of this PSA is complicated, and the control of the condensation reaction conditions has a great influence on the product quality.

Thus, there is always a need to obtain a silicone-based pressure-sensitive adhesive composition having excellent comprehensive properties suitable for medical applications.

### Summary of the invention

The present invention has solved the problems of prior art.

The present invention relates to a medical silicone pressure-sensitive adhesive composition comprising:
- at least one organopolysiloxane A comprising, per molecule, at least two C₂ to C₆ alkenyl radicals each bonded to a silicon atom,
- at least one organopolysiloxane resin **B** comprising OH group(s) bonded to silicone atoms,
- at least one organopolysiloxane extender **CE** having exactly two terminal-hydrogen atom bonded to silicone atoms,
- optionally at least one organopolysiloxane crosslinker **XL** having at least three hydrogen atoms bonded to silicone atoms,
- at least one hydrosilylation catalyst **D**,
- at least one solvent **E**, and
- optionally at least one hydrosilylation inhibitor **F**,
wherein the organopolysiloxanes **A**, **CE** and **XL** are selected such that the molar ratio RHAlk = tH/tAlk is comprised from 2 to 5; and the ratio nH^{XL}/nH^{CE} is below 0.10, with:
- tH = number of moles of hydrogen atom directly bonded to a silicon atom of the organopolysiloxanes **CE** and **XL**;
- tAlk = number of moles of alkenyl directly bonded to a silicon atom of the organopolysiloxane **A**;
- nH^{XL} = number of moles of hydrogen atom directly bonded to a silicon atom of the organopolysiloxane **XL**;
- nH^{CE} = number of moles of hydrogen atom directly bonded to a silicon atom of the organopolysiloxane **CE**.

The medical silicone pressure-sensitive adhesive composition according to the present invention is a precursor of a silicone pressure-sensitive adhesive **G** and is crosslinkable by hydrosilylation.

The present invention further relates to a method for manufacturing a skin-adhesive article, comprising the steps of coating, continuously or non-continuously, a substrate on at least one of its two faces with a medical silicone pressure-sensitive adhesive composition as defined above, and crosslinking said medical silicone pressure-sensitive adhesive composition into a silicone pressure-sensitive adhesive **G.**

Another object of the present invention is a skin-adhesive article comprising a substrate coated, continuously or non-continuously, on at least one of its two faces with a silicone pressure-sensitive adhesive **G** obtained by crosslinking a medical silicone pressure-sensitive adhesive composition as defined above.

It has been discovered that a medical silicone pressure-sensitive adhesive, providing excellent properties suitable for medical applications, can be obtained from the silicone pressure-sensitive adhesive composition as defined above, which is free of, or contains a very low amount of, organopolysiloxane crosslinker **XL**, when the molar ratio RHAlk is comprised from 2 to 5.

Suitable properties for a medical silicone pressure-sensitive adhesive are, for example, excellent overall properties of easy to tear, invisible residual on skin, good anchorage on substrate, good peel adhesion, good tack, suitable release force and acceptable reposition property.

### Detailed description of the invention

All the viscosities under consideration in the present description correspond to a "Newtonian" dynamic viscosity magnitude at 25°C, i.e. the dynamic viscosity which is measured, in a manner that is known per se, with a Brookfield viscometer at a shear rate gradient that is low enough for the measured viscosity to be independent of the rate gradient.

The consistency or penetrability of a gum is typically determined at 25°C by means of a penetrometer of PNR12 type or equivalent model which makes it possible to apply, to the sample, a cylindrical head under standardized conditions. The penetrability of a gum is the depth, expressed in tenths of a millimeter, to which a calibrated cylinder penetrates into the sample over one minute. The following method is given for example: A sample of gum is introduced into an aluminum receptacle with a diameter of 40 mm and with a height of 60 mm. The cylindrical head, made of bronze or of brass, measures 6.35 mm in diameter and 4.76 mm in height and is carried by a metal rod with a length of 51 mm and with a diameter of 3 mm which fits the penetrometer. This rod is ballasted with an excess load of 100 g. The total weight of the assembly is 151.8 g, including 4.3 g for the cylindrical part and its support rod. The receptacle containing the sample of gum is placed in the bath thermostatically controlled at 25 ± 0.5°C, for at least 30 min. The measurement is carried out by following the instructions of the manufacturer. The values of the depth (V), in tenths of a millimeter, and of the time (t), in seconds, to reach this depth are shown on the device. The penetrability is equal to 60 V/t, expressed in tenths of a millimeter per minute.

In the present description, use is made, in order to describe the polyorganosiloxanes, of the nomenclature known in the field of silicones and which uses, in order to describe siloxy units, the following letters: M, D, T and Q. The letter M represents the monofunctional unit of formula (R¹)₃SiO_{1/2}, the silicon atom being connected to just one oxygen atom in the polymer comprising this unit. The letter D means a difunctional unit (R¹)₂SiO_{2/2} in which the silicon atom is connected to two oxygen atoms. The letter T represents a trifunctional unit of formula (R¹)SiO_{3/2}, in which the silicon atom is connected to three oxygen atoms. The letter Q represents a trifunctional unit of formula SiO_{4/2} in which the silicon atom is connected to four oxygen atoms. The symbol R¹ represents a radical. The M, D and T units can be functionalized. Reference is then made to M, D and T units, while specifying the specific radicals.

The component A may be at least two C₂ to C₆ alkenyls substituted silicone polymer used as a backbone in the composition.

Preferably, the at least one organopolysiloxane **A** may comprise:
(i) at least two siloxyl units of formula (**A1**):

   (Y)ₐ(Z)_{b}SiO_{(4-(a+b))/2} (**A1**)

   in which:
   - Y represents a monovalent radical containing from 2 to 6 carbon atoms, having at least two alkenyl group;
   - Z represents a monovalent radical containing from 1 to 20 carbon atoms and not comprising an alkenyl group;
   - a and b represent integers, a being 1, 2 or 3, b being 0, 1 or 2 and (a+b) being 1, 2 or 3;
(ii) and may optionally comprise other siloxyl units of formula (**A2**):

   (Z)_{c}SiO_{(4-c)/2} (**A2**)

   in which:
   - Z has the same meaning as above, and
   - c represents an integer which is 1, 2 or 3.

According to the invention, in the definition of the organopolysiloxane **A** in the formula (**A1**), the symbol a can preferably be equal to 1 or 2, and even more preferentially 1. Furthermore, in formula (**A1**) and in formula (**A2**), the symbol Z may preferentially represent a monovalent radical selected from the group formed by an alkyl group containing 1 to 8 carbon atoms, optionally substituted with at least one halogen atom, and a C₆ to C₁₀ aryl group. Z may advantageously represent a monovalent radical selected from the group formed by: methyl, ethyl, propyl, 3,3,3-trifluoropropyl, xylyl, tolyl and phenyl. In addition, in formula (**A1**), the symbol Y may advantageously represent a radical selected from the group consisting of vinyl, propenyl, 3-butenyl and 5-hexenyl. Preferably, the symbol Y is a vinyl and the symbol Z is a methyl.

The organopolysiloxane **A** may have a linear or branched structure, preferably a linear structure. When it is linear organopolysiloxanes, it can essentially consist:
- of siloxyl units "D" selected from the units of formulae (Y)₂SiO_{2/2}, (Y)(Z)SiO_{2/2} and (Z)₂SiO_{2/2}; and
- of siloxyl units "M" selected from the units of formulae (Y)₃SiO_{1/2}, (Y)₂(Z)SiO_{1/2}, (Y)(Z)₂SiO_{1/2} and (Z)₃SiO_{2/2},
in which formulae, the symbols Y and Z are as defined above.

Preferably, the linear organopolysiloxane **A** has a polymerization degree in a range of 2000 to 10000, more preferably from 2000 to 8000 and more preferably from 2000 to 5000.

By way of example of units "D", mention may be made of dimethylsiloxy, methylphenylsiloxy, methylvinylsiloxy, methylbutenylsiloxy, methylhexenylsiloxy, methyldecenylsiloxy and methyldecadienylsiloxy groups.

By way of example of units "M", mention may be made of trimethylsiloxy, dimethylphenylsiloxy, dimethylvinylsiloxy and dimethylhexenylsiloxy groups.

The organopolysiloxane **A**, in particular when it is linear, may be a polymer preferably having a weight average molecular weight Mw between 260,000 g/mol and 1,000,000 g/mol, preferably between 400,000 g/mol and 1,000,000 g/mol, and more preferably between 400,000 g/mol and 900,000 g/mol.

As examples of organopolysiloxane **A** that are of use, mention may be made of:
- polydimethylsiloxanes comprising dimethylvinylsilyl end groups;
- poly(methylphenylsiloxane-co-dimethylsiloxane)s comprising dimethylvinylsilyl end groups;
- poly(vinylmethylsiloxane-co-dimethylsiloxane)s comprising dimethylvinylsilyl end groups;
- poly(dimethylsiloxane-co-vinylmethylsiloxane)s comprising trimethylsilyl end groups.

The organopolysiloxanes **A** which are polydimethylsiloxanes comprising dimethylvinylsilyl end groups having a weight average molecular weight Mw of between 260,000 g/mol and 1,000,000 g/mol, and preferably of between 400,000 g/mol and 900,000 g/mol, are particularly advantageous. The organopolysiloxanes **A** which are particularly advantageous are those of formula M^{Vi}DₐM^{Vi} in which:
- M^{Vi} = siloxyl unit of formula: (vinyl)(CH₃)₂SiO_{1/2}
- D = siloxyl unit of formula: (CH₃)₂SiO₂₂, and
- a is a number between 2000 and 6000, and preferably between 3000 and 5500.

The organopolysiloxane **A** may be used in an amount from 15wt% to 45wt%, preferably from 20wt% to 40wt% based on the total amount of components **A**+**B**+**XL**+**CE**. According to an embodiment, the organopolysiloxane **A** has an alkenyl content between 0.001wt% to 0.5wt%, preferably 0.005wt% to 0.025wt%, more preferably 0.008wt% to 0.018wt%, based on the total weight of the organopolysiloxane **A.**

Preferably, the organopolysiloxane **A** may be selected from dimethylvinyl-terminated polydimethylsiloxane, dimethylvinyl-terminated polydimethylmethylvinylsiloxane, trimethylterminated polydimethylmethylvinylsiloxane, more preferably selected from dimethylvinylterminated polydimethylsiloxane.

According to one embodiment, the organopolysiloxane **A** is an organopolysiloxane gum having a consistency at 25°C of between 200 mm/10 and 2000 mm/10, preferably between 300 mm/10 and 1800 mm/10, more preferably between 500 mm/10 and 1500 mm/10.

According to one embodiment, the organopolysiloxane **A** is an organopolysiloxane gum having a weight average molecular weight Mw between 260,000 g/mol and 1,000,000 g/mol, preferably between 400,000 g/mol and 1,000,000 g/mol, and more preferably between 400,000 g/mol and 900,000 g/mol. The weight-average molecular weight Mw is determined by gel permeation chromatography with polystyrene as standard.

According to one embodiment, the organopolysiloxane **A** is an organopolysiloxane gum exhibiting a viscosity greater than 600,000 mPa.s at 25°C, preferably greater than 1,000,000 mPa.s at 25°C.

According to another embodiment, the medical silicone pressure-sensitive adhesive composition according to the invention comprises at least two organopolysiloxanes **A**, in which the first organopolysiloxane **A'** is the gum as defined above, and the second organopolysiloxane **A"** is an oil having a dynamic viscosity of 10 mPa.s to 500,000 mPa.s at 25°C, preferably 100 mPa.s to 100,000 mPa.s at 25°C, more preferably 10 000 mPa.s to 100,000 mPa.s at 25°C. The organopolysiloxane **A"** may be linear or branched, and may have alkenyl content of 0.05wt% to 0.5wt%, based on the total weight of component **A"**.

The organopolysiloxane resin **B** comprising hydroxyl groups bonded to Si atom can be selected from conventional organopolysiloxane resins, among which may be mentioned organosilicon resins prepared by cohydrolysis and cocondensation of chlorosilanes selected from the group consisting of those of formulae (R²)₃SiCl, (R²)₂Si(Cl)₂, R²Si(Cl)₃ and Si(Cl)₄. These resins are branched organopolysiloxane oligomers or polymers which are well known and which are commercially available. They exhibit, in their structure, at least two different siloxyl units selected from those of formulae (R²)₃SiO_{1/2} (M unit), (R²)₂SiO_{2/2} (D unit), R²SiO_{3/2} (T unit) and SiO_{4/2} (Q unit), at least one of these units being a T or Q unit. The R² radicals are distributed so that the resins comprise from approximately 0.8 to 1.8 R² radicals per silicon atom. Furthermore, these resins are not completely condensed and contain OH groups. The R² radicals are identical or different and are selected from linear or branched C₁-C₆ alkyl radicals, C₂-C₄ alkenyl radicals, phenyl or 3,3,3-trifluoropropyl. Mention may be made, for example, as alkyl R² radicals, of the methyl, ethyl, isopropyl, tert-butyl and n-hexyl radicals and, as alkenyl radicals, of the vinyl or allyl groups. Preferably, the R² radicals are methyl or hydroxyl groups.

According to a specific embodiment, the organopolysiloxane resin **B** comprising hydroxyl groups is selected from the group consisting of:
a) hydroxylated silicone resins of MQ^{(OH)} type which are copolymers comprising M and Q^{(OH)} siloxy units of following formulae:
   - M = R³R⁴R⁵SiO_{1/2}, and
   - Q^{(OH)} = (OH)SiO_{3/2},
   with optionally the presence of siloxy unit Q = SiO_{4/2};
b) hydroxlyated silicone resins of MD^{Vi}Q^{(OH)} type which are copolymers comprising M, D^{Vi} and Q^{(OH)} siloxy units of following formulae:
   - M = R³R⁴R⁵SiO_{1/2},
   - D^{Vi} = (Vi)(R³)SiO_{2/2}, and
   - Q^{(OH)} = (OH)SiO_{3/2},
   with optionally the presence of siloxy unit Q = SiO_{4/2};
c) hydroxlyated silicone resins of MM^{Vi}Q^{(OH)} type which are copolymers comprising M, M^{Vi} and Q^{(OH)} siloxy units of following formulae:
   - M = R³R⁴R⁵SiO_{1/2},
   - M^{Vi} = (Vi)(R³)(R⁴)SiO_{2/2}, and
   - Q^{(OH)} = (OH)SiO_{3/2},
   with optionally the presence of siloxy unit Q = SiO_{4/2};
d) hydroxlyated silicone resins of MDT^{(OH)}T type which are copolymers comprising M, D, T^{(OH)} and T siloxy units of following formulae:
   - M = R³R⁴R⁵SiO_{1/2},
   - D = R³R⁴SiO_{2/2},
   - T^{(OH)} = (OH)R³SiO_{2/2},
   - T = R³SiO_{3/2}, and
e) hydroxlyated silicone resins of DT^{(OH)}T type which are copolymers comprising D, T^{(OH)} and T siloxy units of following formulae:
   - D = R³R⁴SiO_{2/2},
   - T^{(OH)} = (OH)R³SiO_{2/2},
   - T = R³SiO_{3/2}, and
   in which formulae the symbol Vi = a vinyl group and the symbols R³, R⁴ and R⁵ are selected, independently of one another, from:
   - the linear or branched alkyl groups having from 1 to 8 carbon atoms and optionally substituted by one or more halogen atoms, and preferably selected from the group consisting of the methyl, ethyl, isopropyl, tert-butyl and n-hexyl groups, and
   - aryl or alkylaryl groups having from 6 to 14 carbon atoms inclusive, and preferably selected from the group consisting of the phenyl, xylyl and tolyl groups.

According to a preferred embodiment, mention may be made, as examples of organopolysiloxane resin **B**, selected from at least one of MQ resins, MDQ resins, DT resins and MDT resins, it being possible for the OH groups to be carried by the Q and/or T units.

According to another preferred embodiment, the organopolysiloxane resin **B** is a hydroxylated silicone resin of MQ^{(OH)}, MQQ^{(OH)} or MM^{Vi}Q^{(OH)} type and contains from 0.1wt% to 4wt%, preferably from 0.3wt% to 2.0wt%, more preferably from 0.5wt% to 1.5wt% of hydroxyl group with respect to the dry weight of organopolysiloxane resin **B.** Preferably, the ratio of M units and Q units is from 0.5 to 1.2, preferably from 0.6 to 0.9. Component **B** may have a weight average molecular weight Mw of from 3000 g/mol to 12000 g/mol, preferably from 4000 g/mol to 11000 g/mol.

In this composition of the present invention, the organopolysiloxane resin **B** may be used as a tackifier.

The organopolysiloxane resin **B** may be used in an amount from 25wt% to 80wt%, preferably from 40wt% to 60wt%, based on the total amount of the components **A**+**B**+**XL**+**CE.**

Preferably, the weight ratio between the component **B** and the component **A** may be from 0.8 to 2.5, preferably 1.0 to 2.0.

The component **CE**, also called "chain extender" or just "extender", is an organopolysiloxane having exactly two terminal-hydrogen atoms bonded to Si.

For example, the organohydrosiloxane extender **CE** according to the present invention may comprise:
- two siloxyl end units, which may be identical or different, of formula (**CE-1**):

   (H)(R⁶)₂SiO_{1/2} (**CE-1**)

   in which:
   the symbol R⁶ corresponds to a C₁ to C₈ alkyl group or to a C₆ to C₁₀ aryl group, and the symbol H represents a hydrogen atom;
- at least one siloxyl unit of formula (**CE-2**):

   (R)₂SiO₂₂ (**CE-2**)

   in which the radical R⁷ corresponds to a C₁ to C₈ alkyl group or a C₆ to C₁₀ aryl group,
   with the condition according to which the organopolysiloxane **CE** contains two hydrogen atoms each one bonded to a different silicon atom per polymer, and preferably the organopolysiloxane **CE** contains, per polymer, two siloxyl units of formula (**CE-1**) and at least one siloxyl unit of formula (**CE-2**).

As examples of organopolysiloxane **CE**, mention may be made of polydimethylsiloxanes comprising dimethylhydrosilyl end groups having a dynamic viscosity at 25°C of between 1 mPa.s and 1000 mPa.s, preferably of between 5 mPa.s and 500 mPa.s, even more preferentially of between 5 mPa.s and 300 mPa.s. Particularly advantageous organopolysiloxanes **CE** are of formula M^{H}DₓM^{H} in which:
- M^{H} = siloxyl unit of formula: (H)(CH₃)₂SiO_{1/2}
- D = siloxyl unit of formula: (CH₃)₂SiO₂₂, and
- x is an integer between 1 and 200, preferably between 1 and 150 and even more preferentially between 3 and 120.

The organopolysiloxane **CE** is described as "chain extender" since it has the presumed effect of increasing the mesh size of the network during the crosslinking when the SiH reactive functions are at the chain end.

The component **CE** may have a dynamic viscosity at 25°C of 1 mPa.s to 1000 mPa.s, preferably 5 mPa.s to 500 mPa.s, more preferably 5 mPa.s to 300 mPa.s.

The component CE may have Si-H content of 0.2wt/0 to 10wt%, preferably 0.3wt% to 8.0wt%, more preferably 0.4wt% to 6.0wt%, based on the total weight of component **CE.**

Preferably, the organopolysiloxane extender **CE** may be dimethylhydrogen-terminated polydimethylsiloxane.

The organopolysiloxane extender **CE** may be used in an amount from 5wt% to 20wt%, based on the total amount of the components **A**+**B**+**XL**+**CE**.

As distinct from the chain extender **CE**, the component **XL**, also called "crosslinker", is an organopolysiloxane having at least three hydrogen atoms bonded to Si. The organopolysiloxane crosslinker **XL** is an optional component of the composition according to the present invention. When the organohydrosiloxane crosslinker **XL** is present, it can be as disclosed below:
For example, the organohydrosiloxane crosslinker **XL** according to the present invention may comprise:
- at least three siloxyl units of formula (**XL-1**):

   (H) (L)ₑ SiO_{(3-e)/2} (**XL-1**)

   in which the symbol H represents a hydrogen atom, the symbol L represents an alkyl having from 1 to 8 carbon atoms inclusive or a C₆ to C₁₀ aryl, and the symbol e is equal to 0, 1 or 2; and
- optionally other siloxyl units of formula (**XL-2**):

   (L)_{g} SiO_{(4-g)/2} (**XL-2**)

   in which the symbol L represents an alkyl having from 1 to 8 carbon atoms inclusive or a C₆ to C₁₀ aryl and the symbol g is equal to 0, 1, 2 or 3, and
   with the condition according to which the organopolysiloxane **XL** contains between 0.5% and 15.0% by weight of Si-H function per polymer, preferably between 1.0% and 12.5% by weight of Si-H function per polymer, and even more preferentially between 1.5% and 10.0% by weight of Si-H function per polymer.

As organopolysiloxane **XL** which has a crosslinking function and which is of use according to the invention, mention may be made of those of formulae M^{H}DₓD^{H}_{w}M^{H}, M^{H}DₓD^{H}_{y}M and MDₓD^{H}zM, in which formulae:
- M^{H} = siloxyl unit of formula: (H)(CH₃)₂SiO_{1/2}
- D^{H} = siloxy unit of formula: (H)(CH₃)SiO_{2/2}
- D = siloxyl unit of formula: (CH₃)₂SiO₂₂, and
- M = siloxyl unit of formula: (CH₃)₃SiO_{1/2},
with:
x is a number between 0 and 500, preferably between 2 and 250 and even more preferentially between 5 and 80;
w is a number between 1 and 500, preferably between 1 and 250 or between 1 and 100 and even more preferentially between 1 and 70;
y is a number between 2 and 500, preferably between 3 and 250 or between 2 and 100 and even more preferentially between 2 and 70; and
z is a number between 3 and 500, preferably between 3 and 250 or between 3 and 100 and even more preferentially between 3 and 70, and
comprising between 0.5% and 15.0% by weight of Si-H function per polymer, preferably between 1.0% and 12.5% by weight of Si-H function per polymer, and even more preferentially between 1.5% and 10.0% by weight of Si-H function per polymer.

The component **XL** may have a dynamic viscosity of 40 mPa.s to 1000 mPa.s at 25°C, preferably 50 mPa.s to 750 mPa.s at 25°C, more preferably 60 mPa.s to 500 mPa.s at 25°C. If the viscosity of the component **XL** is lower than 40 mPa.s at 25°C, the cohesion of cured PSA is too strong to tear apart easily, and strong cohesion causes so weak adhesion to the substrate that the cured PSA layer would leave piece residual on the skin when it is peeled off.

The component **XL** may have Si-H content of 0.5wt/0 to 15wt%, preferably 1.0wt% to 12.5wt%, more preferably 1.5wt% to 10.0wt%, based on the total weight of component **XL.**

Preferably, the organopolysiloxane crosslinker **XL** may be trimethylsiloxy-terminated polymethylhydrogensiloxane, or dimethylhydrogen-terminated polymethylhydrogensiloxane.

The contents of the organopolysiloxanes **A**, **CE** and **XL** in the composition according to the present invention are selected according two molar ratios:
- the ratio RHAlk = tH/tAlk, and
- the ratio nHXL/nHCE;
wherein:
- tH = number of moles of hydrogen atom directly bonded to a silicon atom of the organopolysiloxanes **CE** and **XL**;
- tAlk = number of moles of alkenyl directly bonded to a silicon atom of the organopolysiloxane **A**;
- nH^{XL} = number of moles of hydrogen atom directly bonded to a silicon atom of the organopolysiloxane **XL**;
- nH^{CE} = number of moles of hydrogen atom directly bonded to a silicon atom of the organopolysiloxane **CE.**

According to the present invention, the molar ratio RHAlk is comprised from 2 to 5, preferably from 2.5 to 4. According to the present invention, the ratio nH^{XL}/nH^{CE} is below 0.10, preferably from 0 to 0.08, more preferably from 0 to 0.05. According to a preferred embodiment, the composition according to the invention is free of, or substantially free of, organohydrosiloxane crosslinker **XL.**

As hydrosilylation catalyst **D** that is useful according to the invention, mention may be made of the compounds of a metal belonging to the group of platinum which is well known to the person skilled in the art. The metals of the platinum group are those known as platinoids, a name which groups together, in addition to platinum, ruthenium, rhodium, palladium, osmium and iridium. The compounds of platinum and of rhodium are preferably used. Use may in particular be made of the complexes of platinum and of an organic product described in patents US 3,159,601, US 3,159,602 and US 3,220,972 and European patents EP 0 057 459, EP 0 188 978 and EP 0 190 530, and the complexes of platinum and of vinyl organosiloxanes described in patent US 3,419,593. The catalyst generally preferred is platinum. By way of examples, mention may be made of black platinum, chloroplatinic acid, a chloroplatinic acid modified with an alcohol, a complex of chloroplatinic acid with an olefin, an aldehyde, a vinylsiloxane or an acetylenic alcohol, among others. The Karstedt solution or complex, as described in patent US 3,775,452, chloroplatinic acid hexahydrate or a platinum catalyst comprising carbene ligands is preferred.

Preferably, the component **D** is a solution of platinum complex in vinyl-terminated polydimethylsiloxane.

According to one embodiment of the invention, the solvent **E** is selected from the group consisting of: aliphatic C₆ to C₁₆ hydrocarbons, polydimethylsiloxanes comprising a trimethylsilyl end group having a viscosity of 0.65 mPa.s to 5 mPa.s at 25°C, cyclic polydimethylsiloxanes, (3-octyl)heptamethyltrisiloxane, toluene, xylene, a C₁ to C₈ alkylester, a C₂ to C₄ carboxylic acid and their mixtures.

In particular, the solvent **E** is at least one solvent. The solvent **E** is at least one medical approved solvent. For example, it is selected from toluene, xylene, heptane, ethyl acetate, more preferably ethyl acetate for healthcare application.

The amount of solvent **E** is from 20wt% to 70wt%, preferably from 30wt% to 60wt%, based on the total weight of composition.

Hydrosilylation inhibitor **F** can be used in the present composition.

Mention may be made, as example of inhibitor of the hydrosilylation reaction of use according to the invention, of that selected from α-acetylenic alcohols, α, α'-acetylenic diesters, ene-yne conjugated compounds, α -acetylenic ketones, acrylonitriles, maleates, fumarates and the mixtures of these. These compounds, capable of performing the role of hydrosilylation inhibitor, are well known to the person skilled in the art. They can be used alone or as mixtures.

An inhibitor of α -acetylenic alcohol type can be selected from the compounds of following formula (F1):

(R⁸)(R⁹)C(OH)-C≡CH (F1)

in which:
- the R⁸ group represents an alkyl group, a cycloalkyl group, a (cycloalkyl)alkyl group, a C₆ to C₁₀ aryl group or a C₇ to C₁₈ arylalkyl group,
- the R⁹ group represents a hydrogen atom, an alkyl group, a cycloalkyl group, a (cycloalkyl)alkyl group, a C₆ to C₁₀ aryl group or a C₇ to C₁₈ arylalkyl group,
- or else R⁸ and R⁹ constitute, together with carbon atom to which they are bonded, a 5-, 6-, 7- or 8-membered aliphatic ring optionally substituted one or more times.

According to the formula (F1):
- the term "alkyl" is understood to mean a saturated hydrocarbon chain containing from 1 to 20 carbon atoms, preferably from 1 to 8 carbon atoms. An alkyl group can be selected from the group consisting of methyl, ethyl, isopropyl, n-propyl, tert-butyl, isobutyl, n-butyl, n-pentyl, isoamyl and 1,1-dimethylpropyl;
- the term "cycloalkyl" is understood to mean, according to the invention, a saturated monocyclic or polycyclic, preferably monocyclic or bicyclic, hydrocarbon group containing from 3 to 20 carbon atoms, preferably from 5 to 8 carbon atoms. When the cycloalkyl group is polycyclic, the multiple cyclic nuclei can be attached to one another via a covalent bond and/or via a spirane atom and/or be condensed to one another. A cycloalkyl group can be selected from the group consisting of cyclopropyl, cyclobutyl, cyclopentyl, cyclohexyl, cycloheptyl, cyclooctyl, adamantane and norbornane;
- the term "(cycloalkyl)alkyl" is understood to mean, according to the invention, a cycloalkyl group as defined above bonded to an alkyl group as also defined above;
- the term "aryl" is understood to mean, according to the invention, an aromatic hydrocarbon group containing from 6 to 10 carbon atoms which is monocyclic or polycyclic. An aryl group can be selected from the group consisting of phenyl, naphthyl and anthracenyl;
- the term "arylalkyl" is understood to mean, according to the invention, an aryl group as defined above bonded to an alkyl group also as defined above.

According to a preferred embodiment, in the formula (F1), R⁸ and R⁹ constitute, together with a carbon atom to which they are bonded, an unsubstituted 5-, 6-, 7- or 8-membered aliphatic ring. According to another preferred embodiment, R⁸ and R⁹, which are identical or different, represent, independently of one another, a monovalent C₁ to C₁₂, preferably C₁ to C₆, alkyl group.

An inhibitor with an α-acetylenic alcohol of use according to the invention can be selected from the group consisting of the following compounds: 1-ethynyl-1-cyclopentanol; 1-ethynyl-1-cyclohexanol (also known as ECH); 1-ethynyl-1-cycloheptanol; 1-ethynyl-1-cyclooctanol; 3-methyl-1-butyn-3-ol (also known as MBT); 3-methyl-1-pentyn-3-ol; 3-methyl-1-hexyn-3-ol; 3-methyl-1-heptyn-3-ol; 3-methyl-1-octyn-3-ol; 3-methyl-1-nonyn-3-ol; 3-methyl-1-decyn-3-ol; 3-methyl-1-dodecyn-3-ol; 3-methyl-1-pentadecyn-3-ol; 3-ethyl-1-pentyn-3-ol; 3-ethyl-1-hexyn-3-ol; 3-ethyl-1-heptyn-3-ol; 3,5-dimethyl-1-hexyn-3-ol; 3-isobutyl-5-methyl-1-hexyn-3-ol; 3,4,4-trimethyl-1-pentyn-3-ol; 3-ethyl-5-methyl-1-heptyn-3-ol; 3,6-diethyl-1-nonyn-3-ol; 3,7,11-trimethyl-1-dodecyn-3-ol (also known as TMDDO); 1,1,-diphenyl-2-propyn-1-ol; 3-butyn-2-ol; 1-pentyn-3-ol; 1-hexyn-3-ol; 1-heptyn-3-ol; 5-methyl-1-hexyn-3-ol; 4-ethyl-1-octyn-3-ol and 9-ethynyl-9-fluorenol.

An inhibitor of α, α'-acetylenic diester type can be selected from the compounds of following formula (F2): 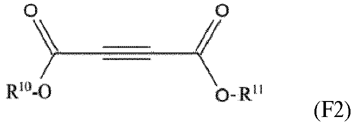 in which the R¹⁰ and R¹¹ groups, which are identical or different, represent, independently of each other, an alkyl group, a cycloalkyl group, a (cycloalkyl)alkyl group, a C₆ to C₁₀ aryl group, a C₇ to C₁₈ arylalkyl group or a silyl group.

The term "silyl" is understood to mean, according to the invention, a group of formula -SiR₃ in which each R symbol independently represents an alkyl group containing from 1 to 20 carbon atoms, preferably from 1 to 8 carbon atoms. A silyl group can be, for example, the trimethylsilyl group.

According to a specific embodiment, in the formula (F2), R¹⁰ and R¹¹, which are identical or different, represent, independently of each other, a C₁ to C₁₂, preferably C₁ to C₆, alkyl group or the trimethylsilyl group. An inhibitor which is an α, α'-acetylenic diester of use according to the invention can be selected from the group consisting of the following compounds: dimethyl acetylenedicarboxylate (DMAD), diethyl acetylenedicarboxylate, di(tert-butyl) acetylenedicarboxylate and bis(trimethylsilyl) acetylenedicarboxylate.

An inhibitor of ene-yne conjugated compound type can be selected from the compounds of following formula (F3): 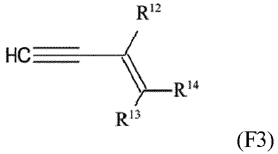 in which:
- the R¹², R¹³ and R¹⁴ groups represent, independently of one another, a hydrogen atom, an alkyl group, a cycloalkyl group, a (cycloalkyl)alkyl group, a C₆ to C₁₀ aryl group or a C₇ to C₁₈ arylalkyl group,
- or also at least two groups from the R¹², R¹³ and R¹⁴ groups constitute, together with the carbon atom or atoms to which they are bonded, a 5-, 6-, 7- or 8-membered aliphatic ring optionally substituted one or more times.

According to a specific embodiment, the R¹², R¹³ and R¹⁴ groups represent, independently of one another, a hydrogen atom, a C₁ to C₁₂, preferably C₁ to C₆, alkyl group or a C₆ to C₁₀ aryl group. An inhibitor which is an ene-yne conjugated compound of use according to the invention can be selected from the group consisting of the following compounds: 3-methyl-3-penten-1-yne; 3-methyl-3-hexen-1-yne; 2,5-dimethyl-3-hexen-1-yne; 3-ethyl-3-buten-1-yne; and 3-phenyl-3-buten-1-yne. According to another specific embodiment, two groups selected from the R¹², R¹³ and R¹⁴ groups constitute, together with the carbon atom or atoms to which they are bonded, an unsubstituted 5-, 6-, 7- or 8-membered aliphatic ring and the remaining third group represents a hydrogen atom or a C₁ to C₁₂, preferably C₁ to C₆, alkyl group. An inhibitor which is an ene-yne conjugated compound of use according to the invention can be 1-ethynyl-1-cyclohexene.

An inhibitor of α-acetylenic ketone type can be selected from the compounds of following formula (F4): 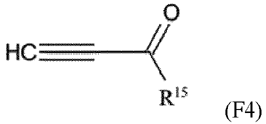 in which: R¹⁵ represents an alkyl group, a cycloalkyl group, a (cycloalkyl)alkyl group, a C₆ to C₁₀ aryl group or a C₇ to C₁₈ arylalkyl group, it being possible for the alkyl, cycloalkyl, (cycloalkyl)alkyl, aryl or arylalkyl groups to be optionally substituted one or more times by a chlorine, bromine or iodine atom.

According to a preferred embodiment, R¹⁵ represents a monovalent C₁ to C₁₂, preferably C₁ to C₆, alkyl group optionally be substituted one or more times by a chlorine or bromine atom, or a cycloalkyl group, or a C₆ to C₁₀ aryl group. An inhibitor which is an α-acetylenic ketone of use according to the invention can be selected from the group consisting of the following compounds: 1-octyn-3-one; 8-chloro-1-octyn-3-one; 8-bromo-1-octyn-3-one; 4,4-dimethyl-1-octyn-3-one; 7-chloro-1-heptyn-3-one; 1-hexyn-3-one; 1-pentyn-3-one; 4-methyl-1-pentyn-3-one; 4,4-dimethyl-1-pentyn-3-one; 1-cyclohexyl-1-propyn-3-one; benzoacetylene and (o-chlorobenzoyl)acetylene.

An inhibitor of acrylonitrile type can be selected from the compounds of following formula (F5): 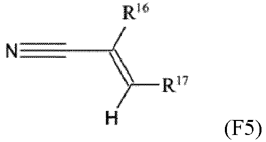 in which: R¹⁶ and R¹⁷ represent, independently of each other, a hydrogen atom, a chlorine, bromine or iodine atom, an alkyl group, a cycloalkyl group, a (cycloalkyl)alkyl group, a C₆ to C₁₀ aryl group or a C₇ to C₁₈ arylalkyl group, it being possible for the alkyl, cycloalkyl, (cycloalkyl)alkyl, aryl or arylalkyl groups to be optionally substituted one or more times by a chlorine, bromine or iodine atom.

An inhibitor which is an acrylonitrile of use according to the invention can be selected from the group consisting of the following compounds: acrylonitrile; methacrylonitrile; 2-chloroacrylonitrile; crotononitrile and cinnamonitrile.

An inhibitor of maleate or fumarate type can be selected from the compounds of following formulae (F6) and (F7): 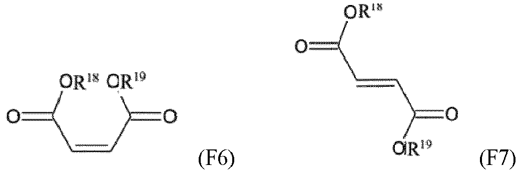 in which: R¹⁸ and R¹⁹, which are identical or different, represent, independently of each other, an alkyl or alkenyl group, a cycloalkyl group, a (cycloalkyl)alkyl group, a C₆ to C₁₀ aryl group or a C₇ to C₁₈ arylalkyl group, it being possible for said alkyl, alkenyl, cycloalkyl, (cycloalkyl)alkyl, aryl and arylalkyl groups to be substituted by an alkoxy group.

The term "alkenyl" is understood to mean, according to the invention, a saturated hydrocarbon chain containing from 2 to 6 carbon atoms and comprising at least one double unsaturation. Preferably, the alkenyl group is selected from the group consisting of a vinyl and an allyl. The term "alkoxy" is understood to mean, according to the formulae (F6) and (F7), an alkyl group as defined above bonded to an oxygen atom. An alkoxy group can be selected from the group consisting of methoxy, ethoxy, propoxy and butoxy.

According to a specific embodiment, R¹⁸ and R¹⁹, which are identical or different, represent, independently of each other, a C₁ to C₁₂, preferably C₁ to C₆, alkyl or alkenyl group optionally substituted by a C₁ to C₆ alkoxy group.

An inhibitor which is a maleate or a fumarate of use according to the invention can be selected from the group consisting of diethyl fumarate, diethyl maleate, diallyl fumarate, diallyl maleate and bis(methoxyisopropyl) maleate.

These inhibitors can be added in an amount by weight of between 1 ppm and 50,000 ppm, with respect to the weight of the total silicone composition, in particular between 10 ppm and 10,000 ppm, preferably between 20 ppm and 2000 ppm and more preferentially still between 800 ppm and 2000 ppm.

The medical silicone pressure-sensitive adhesive composition according to the present invention may be applied to various substrates suitable for medical application. The substrate can be a support of highly varied natures, according to the field of application.

According to a preferred embodiment, the substrate is a woven, nonwoven or knitted textile or a film of plastic. The term "nonwoven" is understood to mean any structure consisting of textile materials, such as fibers, continuous filaments or cut yarns, whatever the nature or the origin thereof, formed into a net by any means and bonded by any means, excluding the intertwining of yarns. Nonwovens are products having the appearance of textiles, are porous, are composed mainly of fibers and are manufactured by processes other than spinning, weaving, knitting or knotting.

According to another preferred embodiment, the substrate is made of plastic. A large variety of plastics can be appropriate for use as substrate according to the invention. Examples comprise: polyvinyl chloride, polypropylene, regenerated cellulose, polyethylene terephthalate (PET) and polyurethane, in particular melt-blown polyurethane. The substrate can be a perforated flexible polyurethane film or a continuous flexible polyurethane film. This flexible polyurethane film can be manufactured from melt-blown polyurethane. When the substrate is a flexible polyurethane film, the thickness will generally be between 5 jam and 600 µm, preferably between 5 µm and 250 µm and more preferably still between 10 µm and 100 µm.

Alternatively, the substrate may be selected from paper, non-woven cloth and elastic cloth.

According to a preferred embodiment, the substrate may be selected from paper, non-woven cloth, elastic cloth or a film of plastic, preferably a film of a plastic selected from the group consisting of polyvinyl chloride, polypropylene, regenerated cellulose, polyethylene terephthalate (PET) and polyurethane.

The person skilled in the art can adjust the medical silicone pressure-sensitive adhesive composition according to the final application. Generally, the medical silicone pressure-sensitive adhesive composition according to the present invention may have a dynamic viscosity at 25°C of 500 mPa.s to 5000 mPa.s, preferably 800 mPa.s to 3000 mPa.s, more preferably 1000 mPa.s to 2500 mPa.s.

The medical silicone pressure-sensitive adhesive composition according to the present invention can be applied or coated to various substrates by any technique well known by the person skilled in the art. As technique for depositing the medical silicone pressure-sensitive adhesive composition according to the present invention, mention may be made, for example, of the coating techniques carried out by a knife, in particular by knife overroll, floating knife and knife over carpet, or by padding, that is to say by squeezing between two rolls, or also by licking roll, rotary machine, reverse roll or transfer, or by spraying. Mention may be made, as other coating technique, of the curtain coating technique. Curtain coating is a process for application of a coating liquid to an article or a support. Curtain coating is characterized by the formation of a freely falling curtain of a coating liquid which falls from the lip of the hopper and, under the effect of gravity, will encounter the article moving through the curtain in order to form a coating. This technique has been widely used in the field of the preparation of multilayer photosensitive silver supports (see, for example, the patents US 3,508,947, US 3,508,947 and EP 537 086).

Then, the medical silicone pressure-sensitive adhesive composition coated on the substrate is crosslinked, for example, at the temperature of 100°C to 160°C, preferably 120°C to 150°C.

Thus, a substrate coated with a silicone pressure-sensitive adhesive **G** is obtained by crosslinking the silicone pressure-sensitive adhesive composition according to the present invention.

Other advantages and features of the present invention will appear on reading the following examples that are given by way of illustration and that are in no way limiting.

### Examples

Probe Tack test: the tack test is carried out by cutting cured PSA lamination into strips of 2.5cm(length)^{∗}2.5cm(width), taking off the release liner from PSA layer; running the Tack test on the PT1000 Probe Tack Tester from Cheminstrument Co. Ltd according to ASTM D2979. Atmosphere condition is 23°C & 50% RH.

Peel Adhesion: the peel adhesion test is carried out by cutting cured PSA lamination into strips of 10cm(length)^{∗}2.5cm(width), taking off the release liner from PSA layer, adhering the PSA layer on a cardpaper of 15cm^{∗}5cm size and laminating them with a 2 kg rubber roller two cycles. Then, the peel test is performed on the PA1000-180 Peel Tester from Cheminstrument Co. Ltd according to FINAT FTM1. Atmosphere condition is 23°C & 50% RH.

Raw materials used in the examples are listed below:
Organopolysiloxane **A**: vinyl-terminated polydimethylsiloxane gum M^{Vi}DₓM^{Vi} Mw=560,000 g/mol, vinyl content=0.015wt%, consistency = 800 mm/10
Organopolysiloxane resin **B**: MₓQ_{y}Q^{(OH)}_{z} resin, Mw=5,500 g/mol, x/(y+z) = 0.9, OH=1.0wt% Extender **CE**: hydrogen-terminated polydimethylsiloxane oil M^{H}D_{y}M^{H}, Viscosity=8.0 mPa.s, Si-H content=5.51wt%
Catalyst **D**: Pt content=0.2wt%, dissolved in vinyl terminated polydimethylsiloxane of 350 mPa.s Solvent E: Ethyl Acetate

### Examples 1 - 5

All components were mixed homogeneously in amounts as shown in the following table.

The obtained composition was coated with blade on a PET film (60-90 g/m²), and then put in a oven at 120°C for 30 min.

**Table 1**

| | **Ex.1** | **Ex.2** | **Ex.3** | **Ex.4** | **Ex.5** |
|---|---|---|---|---|---|
| Organopolysiloxane **A** | 23.93 | 23.70 | 22.59 | 20.64 | 17.62 |
| Organopolysiloxane resin **B** | 36.00 | 35.65 | 33.96 | 31.03 | 26.48 |
| Extender **CE** | 1.51 | 2.46 | 7.07 | 15.07 | 27.53 |
| Catalyst **D** | 1.17 | 1.16 | 1.10 | 1.01 | 0.86 |
| Solvent **E** | 37.39 | 37.03 | 35.28 | 32.25 | 27.51 |

| Molar ratio | | | | | |
|---|---|---|---|---|---|
| RHAlk (= tH/tAlk) | 0.6 | 1.0 | 3.0 | 7.0 | 15.0 |

| Properties | | | | | |
|---|---|---|---|---|---|
| Tack (mJ/cm²) | 51 | 50 | 35 | 35 | 15 |
| Peel (N/cm) | 1.7 | 3.4 | 1.2 | 0.8 | 0.2 |
| Observations | uncured | uncured | tacky | cured / low peel | cured / low peel |

The composition according to the present invention (Ex.3) provided after curing an adhesive having very good properties, which could advantageously be sued as a medical PSA. Below claimed molar ratio RHAlk (Ex.1 and Ex.2), the composition was not fully cured. Above claimed molar ratio RHAlk (Ex.4 and Ex.5), the cured composition lose peel and seemed to be closer to an elastomer.

## Claims

1. A medical silicone pressure-sensitive adhesive composition comprising:
- at least one organopolysiloxane **A** comprising, per molecule, at least two C₂ to C₆ alkenyl radicals each bonded to a silicon atom,
- at least one organopolysiloxane resin **B** comprising OH group(s) bonded to silicone atoms,
- at least one organopolysiloxane extender **CE** having exactly two terminal-hydrogen atom bonded to silicone atoms,
- optionally at least one organopolysiloxane crosslinker **XL** having at least three hydrogen atoms bonded to silicone atoms,
- at least one hydrosilylation catalyst **D**,
- at least one solvent **E**, and
- optionally at least one hydrosilylation inhibitor **F**,
wherein the organopolysiloxanes **A**, **CE** and **XL** are selected such that the molar ratio RHAlk = tH/tAlk is comprised from 2 to 5, and the ratio nH^{XL}/nH^{CE} is below 0.10, with:
- tH = number of moles of hydrogen atom directly bonded to a silicon atom of the organopolysiloxanes **CE** and **XL**;
- tAlk = number of moles of alkenyl directly bonded to a silicon atom of the organopolysiloxane **A**;
- nH^{XL} = number of moles of hydrogen atom directly bonded to a silicon atom of the organopolysiloxane **XL**;
- nH^{CE} = number of moles of hydrogen atom directly bonded to a silicon atom of the organopolysiloxane **CE**.

2. The medical silicone pressure-sensitive adhesive composition according to claim 1, wherein the molar ratio RHAlk is comprised from 2.5 to 4.

3. The medical silicone pressure-sensitive adhesive composition according to claim 1 or claim 2, wherein the ratio nH^{XL}/nH^{CE} is strictly below 0.10, preferably from 0 to 0.08, more preferably from 0 to 0.05.

4. The medical silicone pressure-sensitive adhesive composition according to anyone of claims 1 to 3, wherein the composition is free of, or substantially free of, organohydrosiloxane crosslinker **XL.**

5. The medical silicone pressure-sensitive adhesive composition according to anyone of claims 1 to 4, wherein the organopolysiloxane **A** is selected from dimethylvinyl-terminated polydimethylsiloxane, dimethylvinyl-terminated polydimethylmethylvinylsiloxane, trimethylterminated polydimethylmethylvinylsiloxane, more preferably selected from dimethylvinylterminated polydimethylsiloxane.

6. The medical silicone pressure-sensitive adhesive composition according to anyone of claims 1 to 5, wherein the organopolysiloxane **A** is an organopolysiloxane gum having a consistency at 25°C of between 200 mm/10 and 2000 mm/10, preferably between 300 mm/10 and 1800 mm/10, more preferably between 500 mm/10 and 1500 mm/10.

7. The medical silicone pressure-sensitive adhesive composition according to anyone of claims 1 to 6, wherein the organopolysiloxane **A** is used in an amount from 15wt% to 45wt%, preferably from 20wt% to 40wt% based on the total amount of components **A**+**B**+**XL**+**CE**.

8. The medical silicone pressure-sensitive adhesive composition according to anyone of claims 1 to 7, wherein the organopolysiloxane resin **B** comprising hydroxyl groups is selected from the group consisting of:
a) hydroxylated silicone resins of MQ^{(OH)} type which are copolymers comprising M and Q^{(OH)} siloxy units of following formulae:
- M = R³R⁴R⁵SiO_{1/2}, and
- Q^{(OH)} = (OH)SiO_{3/2},
with optionally the presence of siloxy unit Q = SiO_{4/2};
b) hydroxlyated silicone resins of MD^{Vi}Q^{(OH)} type which are copolymers comprising M, D^{Vi} and Q^{(OH)} siloxy units of following formulae:
- M = R³R⁴R⁵SiO_{1/2},
- D^{Vi} = (Vi)(R³)SiO_{2/2}, and
- Q^{(OH)} = (OH)SiO_{3/2},
with optionally the presence of siloxy unit Q = SiO_{4/2};
c) hydroxlyated silicone resins of MM^{Vi}Q^{(OH)} type which are copolymers comprising M, M^{Vi} and Q^{(OH)} siloxy units of following formulae:
- M = R³R⁴R⁵SiO_{1/2},
- M^{Vi} = (Vi)(R³)(R⁴)SiO_{2/2}, and
- Q^{(OH)} = (OH)SiO_{3/2},
with optionally the presence of siloxy unit Q = SiO_{4/2};
d) hydroxlyated silicone resins of MDT^{(OH)}T type which are copolymers comprising M, D, T^{(OH)} and T siloxy units of following formulae:
- M = R³R⁴R⁵SiO_{1/2},
- D = R³R⁴SiO_{2/2},
- T^{(OH)} = (OH)R³SiO_{2/2},
- T = R³SiO_{3/2}, and
e) hydroxlyated silicone resins of DT^{(OH)}T type which are copolymers comprising D, T^{(OH)} and T siloxy units of following formulae:
- D = R³R⁴SiO_{2/2},
- T^{(OH)} = (OH)R³SiO_{2/2},
- T = R³SiO_{3/2}, and
in which formulae the symbol Vi = a vinyl group and the symbols R³, R⁴ and R⁵ are selected, independently of one another, from:
- the linear or branched alkyl groups having from 1 to 8 carbon atoms and optionally substituted by one or more halogen atoms, and preferably selected from the group consisting of the methyl, ethyl, isopropyl, tert-butyl and n-hexyl groups, and
- aryl or alkylaryl groups having from 6 to 14 carbon atoms inclusive, and preferably selected from the group consisting of the phenyl, xylyl and tolyl groups.

9. The medical silicone pressure-sensitive adhesive composition according to anyone of claims 1 to 8, wherein the organopolysiloxane resin **B** is used in an amount from 25wt% to 80wt%, preferably from 40wt% to 60wt%, based on the total amount of the components **A**+**B**+**XL**+**CE**.

10. The medical silicone pressure-sensitive adhesive composition according to anyone of claims 1 to 9, wherein the organopolysiloxane **CE** is of formula M^{H}DₓM^{H} in which:
- M^{H} = siloxyl unit of formula: (H)(CH₃)₂SiO_{1/2}
- D = siloxyl unit of formula: (CH₃)₂SiO₂₂, and
- x is an integer between 1 and 200, preferably between 1 and 150 and even more preferentially between 3 and 120.

11. The medical silicone pressure-sensitive adhesive composition according to anyone of claims 1 to 10, wherein the solvent **E** is selected from the group consisting of: aliphatic C₆ to C₁₆ hydrocarbons, polydimethylsiloxanes comprising a trimethylsilyl end group having a viscosity of 0.65 mPa.s to 5 mPa.s at 25°C, cyclic polydimethylsiloxanes, (3-octyl)heptamethyltrisiloxane, toluene, xylene, a C₁ to C₈ alkylester, a C₂ to C₄ carboxylic acid and their mixtures, preferably from the group consisting of toluene, xylene, heptane, and ethyl acetate, and more preferably the solvent **E** is ethyl acetate for healthcare application.

12. The medical silicone pressure-sensitive adhesive composition according to anyone of claims 1 to 11, wherein the amount of solvent **E** is from 20wt% to 70wt%, preferably from 30wt% to 60wt%, based on the total weight of composition.

13. The medical silicone pressure-sensitive adhesive composition according to anyone of claims 1 to 12, wherein the substrate is selected from paper, non-woven cloth, elastic cloth or a film of plastic, preferably a film of a plastic selected from the group consisting of polyvinyl chloride, polypropylene, regenerated cellulose, polyethylene terephthalate (PET) and polyurethane.

14. A method for manufacturing a skin-adhesive article, comprising the steps of coating, continuously or non-continuously, a substrate on at least one of its two faces with a medical silicone pressure-sensitive adhesive composition according to any one of claims 1 to 13, and crosslinking said medical silicone pressure-sensitive adhesive composition into a silicone pressure-sensitive adhesive **G.**

15. A skin-adhesive article comprising a substrate coated, continuously or non-continuously, on at least one of its two faces with a silicone pressure-sensitive adhesive **G** obtained by crosslinking a medical silicone pressure-sensitive adhesive composition according to anyone of claims 1 to 13.
